# EUROPÄISCHE PATENTSCHRIFT

(11) **EP 2 734 456 B1**
(45) Veröffentlichungstag und Bekanntmachung des Hinweises auf die Patenterteilung: **31.08.2016**
(21) Anmeldenummer: 12766576.8
(22) Anmeldetag: 16.07.2012
(51) Int. Cl.: B65D 75/58

(54) **EINZELDOSISVERPACKUNG FÜR TRANSDERMALE THERAPEUTISCHE SYSTEME ODER FOLIENFÖRMIGE DARREICHUNGSFORMEN**
SINGLE-DOSE PACKAGE FOR TRANSDERMAL THERAPEUTIC SYSTEM OR SHEET-LIKE ADMINISTRATION FORMS
EMBALLAGE DE DOSE INDIVIDUELLE POUR DES SYSTÈMES THÉRAPEUTIQUES TRANSDERMIQUES OU DES FORMES GALÉNIQUES PELLICULAIRES

(30) Priorität: 19.07.2011 DE 102011107939
(43) Veröffentlichungstag der Anmeldung: 28.05.2014
(73) Patentinhaber: LTS Lohmann Therapie-Systeme AG, 56626 Andernach (DE)
(72) Erfinder: HUHN, Ralf, 53639 Königswinter (DE); BOTZEM, Petra, 56626 Andernach (DE); SCHMIDT, Arno, 56242 Marienrachdorf (DE); SCHÜLLER, Tobias, 56743 Mendig (DE)
(86) Internationale Anmeldenummer: PCT/EP2012/002983
(87) Internationale Veröffentlichungsnummer: WO 2013/010659

(56) Entgegenhaltungen:
- DE-A1-102004 047 445
- DE-A1-102004 062 864
- DE-U1-202004 003 781
- US-A1- 2006 023 976
- US-A1- 2009 283 440

## Beschreibung

Die Erfindung betrifft eine Verpackung für einzeldosierte wirkstoffhaltige Filme, umfassend ein oberes Packstoff-Element und ein unteres Packstoff-Element, die mittels einer umlaufenden Siegelfläche bzw. Siegelnaht derart miteinander verbunden sind, dass ein allseitig umschlossener Hohlraum zur Aufnahme des Films gebildet wird, wobei das obere Packstoffelement und das untere Packstoffelement im Bereich der Siegelfläche bzw. der Siegelnaht jeweils mindestens einen Schnitt aufweisen, die deckungsgleich sind, und mindesten ein Schnitt durch eine Falz- oder Knicklinie gekreuzt wird.

Eine Verpackung der vorgenannten Art ist aus der deutschen Offenlegungsschrift DE 10 2009 008 217 A1 bekannt.

Aus der DE 10 2004 047 445 A1 ist eine nicht wiederverschliessbare Verpackung für gesundheitsgefährdende Erzeugnisse insbesondere pharmazeutische Erzeugnisse, umfassend ein erstes Packstoff-Element und eine zweites Packstoff-Element bekannt. Dabei sind die beiden Packstoff-Elemente übereinanderliegend angeordnet und die Verpackung weist mindestens einen ersten Flächenabschnitt auf, an dessen Rand oder Rändern die beiden Packstoff-Elemente lösbar miteinander verbunden sind, wobei zwischen den beiden Packstoff-Elementen mindestens ein allseitig umschlossener Hohlraum zur Aufnahme des Packgutes gebildet wird. Die Verpackung weist mindestens einen zweiten Flächenabschnitt auf, der außerhalb des genannten ersten Flächenabschnitts liegt oder an diesen angrenzt, und an dessen Rand oder Rändern die beiden Packstoff-Elemente lösbar miteinander verbunden sind und wobei zumindest eines der beiden Packstoff-Elemente mit mindestens einer Struktur versehen ist, die innerhalb des zweiten Flächenabschnitts verläuft und die das Einreißen des/der Packstoff-Elements/Elemente ermöglicht.

Aus der DE 10 2006 041 921 A1 ist eine Verpackung für wirkstoffhaltige Filme, die eine Trägerschicht und eine mit dieser lösbar verbundene Deckschicht aufweist, bekannt. Die Verpackung weist dabei in einer paarweisen Anordnung zwei gegenüberliegende, durch einen Steg voneinander getrennten Flächenbereiche auf, innerhalb welcher die Deckschicht nicht mit der Trägerschicht verbunden ist, wodurch zwei voneinander getrennte, allseitig umschlossene Räume zur paarweisen Aufnahme der genannten Filme gebildet werden. Innerhalb des genannten Stegs ist ein weiterer Flächenbereich vorhanden, in welchem die Trägerschicht nicht mit der Deckschicht verbunden ist, wodurch ein allseitig umschlossener Hohlraum gebildet wird und innerhalb des Stegs mindestens eine Perforationslinie vorhanden ist.

Aus der DE 10 2004 062 864 A1 ist ein Folienbehälter mit zwei zur Ausbildung einer Aufnahmekammer für ein Füllgut, insbesondere einer pharmazeutischen Wirkstoffformulierung, umlaufend miteinander verbundenen, insbesondere viereckförmigen Folien, von denen mindestens eine Folie zum Aufreißen mit einer innerhalb des Verbindungsbereichs ausgebildeten Markierung versehen, die nach einem Knicken der Folien freigegeben ist. Dabei verläuft die Markierung mittig zwischen zwei gegenüberliegenden Außenkanten des Folienbehälters.

Aus der DE 20 2004 003 781 U1 ist eine kindersichere Verpackung aus zwei miteinander verbundenen Folien, deren flächiger Verschlussbereich mindestens einen Aufnahmeraum für das Füllgut einschließt, bekannt, wobei eine vom Verschlussbereich vollständig umschlossene physikalische Markierung in Form einer Änderung, insbesondere Schwächung oder Wegnahme eines Abschnitts des Randbereichs vorgesehen ist.

Verpackungen für einzeldosierte wirkstoffhaltige Filme sind auch aus DE 102009008217 A1, WO 2012/084216 A1, US 2009/283440 A1 und US 2006/023976 A1 bekannt.

Bei den bekannten Ausführungen der Verpackung muss der Nutzer einen Teil der Verpackung entlang einer aufgedruckte Markierungslinie umknicken, um anschließend an einer ebenfalls markierten Stelle die Verpackung durch Reißen entlang einer Schwächungslinie zu öffnen. Das Umknicken entlang der aufgedruckten Markierungslinie verändert die Lage einer Struktur oder eines Schnittes im Bereich der Siegelfläche bzw. Siegelnaht der beiden Packstoffelementen an den Rand der Verpackung und ermöglicht so ein aufreißen der Verpackung, um den Inhalt aus dem Hohlraum entnehmen zu können. Die Struktur oder der Schnitt hat eine Länge von ca. 3 bis 5 mm. Die aufgedruckte Markierungslinie kreuzt die Struktur bzw. den Schnitt vorzugsweise rechtwinklig oder unter einem ausgewählten Winkel. Je nach Aufbau und Materialwahl der Packstoffelemente wird beim Umknicken die Länge der Struktur bzw. des Schnitts nicht mittig umgefaltet sondern so, dass zwei unterschiedlich lange Schenkel der Struktur bzw. des Schnitts ausgebildet sind. Im schlimmsten Fall wird die Struktur bzw. der Schnitt gar nicht an den Rand verlagert. Hierdurch wird ein Einreißen der Verpackung erschwert bzw. sogar verhindert, da die/der als Einreißhilfe gedachte Struktur/Schnitt nicht zum Einsatz kommen.

Der Erfindung liegt daher die Aufgabe zugrunde, eine gattungsgemäße Einzeldosisverpackung zu schaffen, welche die oben beschriebenen Ausführungen verbessert, insbesondere deren Handhabung vereinfacht.

Diese Aufgabe wird erfindungsgemäß durch die Verpackung des Anspruchs 1 gelöst.

Ausgestaltungen der Erfindung sind in den Unteransprüchen angegeben.

Die Erfindung sieht somit vor, die äußere Schicht des oberen Packstoffelementes entlang der Falz- oder Knicklinie zu schwächen um ein Umknicken an genau dieser Position zu ermöglichen. Durch die Schwächung wird erreicht, dass die Struktur bzw. der Schnitt genau mittig umgeklappt wird und die Stelle zum Einreißen immer am Rand der Verpackung positioniert ist.

Die Schwächung wird so durchgeführt, dass nur die äußere Schicht des Packstoffelementes verändert bzw. bearbeitet wird. Die nachfolgende Metallfolie wird gar nicht oder nur gering beansprucht. Die Schwächung kann beispielsweise durch perforieren, kerbstanzen, rillen oder durch Laserablation hergestellt werden. Andere Bearbeitungsformen sind aber ausdrücklich erwünscht.

Die äußere Schicht ist vorzugsweise bedruckbar, so dass z.B. Produktkennzeichnungen und Aufreißhilfen angebracht werden können. Die Schwächung kann dabei die gesamte Dicke der äußeren Schicht berücksichtigen oder aber auch nur eine Teilhöhe. Während bei einer Rillung die äußere Schicht verdrängt wird kann z.B. bei der Laserablation das Material bis auf die nachfolgende Metallschicht abgetragen werden.

Eine zweite bzw. bei dreischichtigem Aufbau die mittlere Schicht besteht aus einer Metallfolie, vorzugsweise Aluminium, mit einer Dicke von 9 - 25 Mikrometer. Diese Metallschicht sorgt für die Dichtigkeit der Verpackung gegenüber Feuchtigkeit und Luft..
Die innere Schicht ist eine siegelfähige Kunststoffschicht, wobei die durch diese Schicht erzeugte Siegelnaht nicht wieder geöffnet werden kann.

In einer bevorzugten Ausführungsform besitzen das obere Packstoffelement und das untere Packstoffelement einen identischen Aufbau. Dieses hat den Vorteil, dass nur eine Sorte von Packstoffen zum Einsatz und damit eine Verwechslung ausgeschlossen wird.

Die Verbindung zwischen den beiden Packstoffelementen wird vorzugsweise durch Siegeln oder Schweißen erzeugt. Geeignete Mittel und Verfahren zum Erzeugen der Siegelflächen bzw. der Siegelnähte sind dem Fachmann bekannt. Die Siegelung dient als Diffusions- und Permeationsbarriere und ist im allgemeinen undurchlässig für Wirkstoffe und Luftfeuchtigkeit. Es kommen sowohl Heißsiegelverfahren als auch Kaltsiegelverfahren in Betracht. Als Material für Siegelschichten können z.B. Schmelzkleber, Siegellacke, Siegeldispersionen oder Klebstoffe verwendet werden.

In einer weiteren Ausführungsform sind in der Siegelfläche bzw. der Siegelnaht zwei parallel ausgerichtete Schnitte eingebracht. Diese sind so angeordnet, dass die Verlängerung der Schnitte, also der Risslinien, durch den Hohlraum verlaufen. Werden beide Siegelränder abgerissen, kann der wirkstoffhaltige Film aus dem Hohlraum geschoben werden. Dabei wird die Falz- oder Knicklinie in einer bevorzugten Ausführung so angeordnet, dass beide Schnitte rechtwinklig gekreuzt wird. Beim Umknicken entlang der geschwächten Falz- oder Knicklinie werden dann beide Schnitte an den Rand verlagert und somit zwei Einreißstellen erzeugt.

Weitere Merkmale und Einzelheiten der Erfindung ergeben sich aus den Ansprüchen und der nachfolgenden Beschreibung von in schematischen Zeichnungen dargestellten Ausführungsbeispielen der Erfindung. Es zeigen:
- Fig. 1: in Draufsicht eine Verpackung für wirkstoffhaltige Filme;
- und Fig. 2: in geschnittener Seitenansicht die Verpackung aus Figur 1;

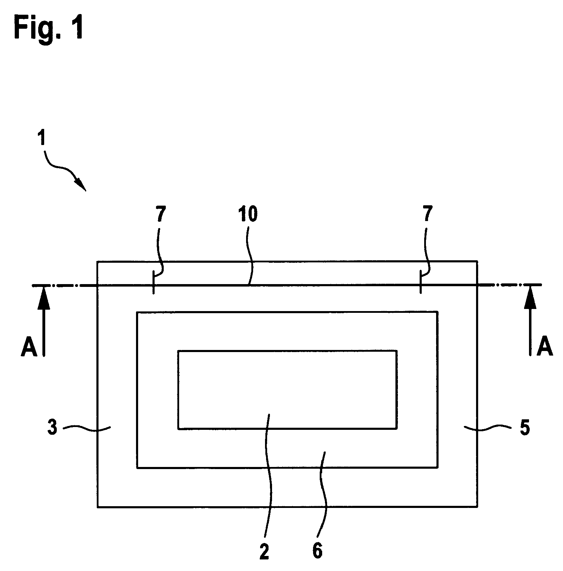

In Fig. 1 ist erfindungsgemäße Verpackung 1 in Draufsicht dargestellt. Bei der Verpackung 1 handelt es sich um einen Siegelrandbeutel aus einem oberen Packstoffelement 3 und einem unteren Packstoffelement 4. Zwischen dem oberen Packstoffelement 3 und dem unteren Packstoffelement 4 ist ein wirkstoffhaltiger Film 2 in einem allseitig umschlossenen Hohlraum 6 angeordnet. Der Hohlraum 6 wird durch eine umlaufende Siegelfläche bzw. Siegelnaht 5 erzeugt, die die beiden Packstoffelemente 3, 4 unlösbar verbindet. In der Siegelfläche bzw. Siegelnaht 5 befindet sich in dem oberen Packstoffelement 3 ein Schnitt 7 und in dem unteren Packstoffelement 4 ein Schnitt 8. Die beiden Schnitte 7 und 8 sind deckungsgleich. Der Schnitt 7 wird durch eine Falz- oder Knicklinie 10 gekreuzt.

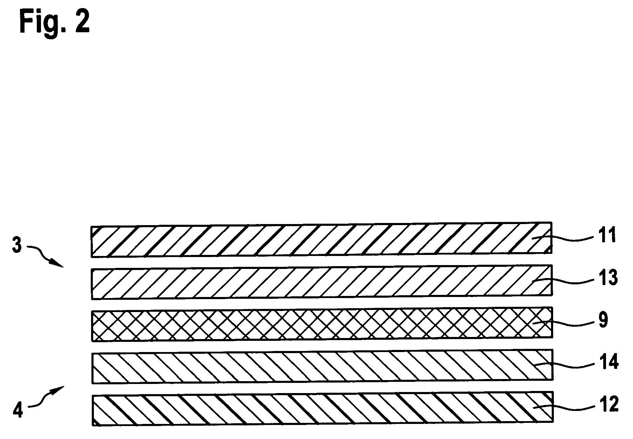

In Fig. 2 ist der Aufbau der Verpackung in geschnittener Darstellung entlang der Linie A - A in Fig. 1 dargestellt. Die einzelnen Teile sind stark vergrößert und getrennt gezeichnet, um den Aufbau zu verdeutlichen. Das obere Packstoffelement 3 besteht mindestens aus einer oberen, äußeren Schicht 11 und einer nachfolgenden oberen Metallschicht 13.

Die untere Packstoffschicht 4 besteht ebenfalls aus einer unteren, äußeren, Schicht 12 und einer nachfolgenden unteren Metallschicht 14.

Die Metallschichten 13, 14 bestehen verzugsweise aus einer Aluminiumfolie mit einer Dicke von 9 bis 25 Mikrometer. Diese Folie sorgt dafür, dass das eingelegte Produkt nicht mit Luft in Berührung kommt und keinerlei Feuchtigkeit ausgesetzt ist.

Mindestens im Bereich der Siegelfläche bzw. der Siegelnaht 5 weist das obere oder das untere Packstoffelement 3, 4 eine siegelfähige Schicht 9 auf. Die siegelfähige Schicht 9 kann aber auch auf beiden, dem oberen und dem unteren, Packstoffelementen 3, 4 angebracht sein. Die durch diese Schicht 9 erzeugte Siegelfläche bzw. Siegelnaht 5 kann nicht wieder getrennt werden.

### Bezugszeichenliste:

- 1: Verpackung
- 2: wirkstoffhaltiger Film
- 3: oberes Packstoffelement
- 4: unteres Packstoffelement
- 5: Siegelfläche bzw. Siegelnaht
- 6: Hohlraum
- 7: Schnitt
- 8: Schnitt
- 9: Siegelschicht
- 10: Falz- oder Knicklinie
- 11: obere, äußere Schicht
- 12: untere, äußere Schicht
- 13: obere Metallschicht
- 14: untere Metallschicht

## Patentansprüche

1. Verpackung (1) für einzeldosierte wirkstoffhaltige Filme (2), umfassend ein oberes Packstoff-Element (3) und ein unteres Packstoff-Element (4), die mittels einer umlaufenden Siegelfläche bzw. Siegelnaht (5) derart miteinander verbunden sind, dass ein allseitig umschlossener Hohlraum (6) zur Aufnahme des Films (2) gebildet wird, wobei
- das obere Packstoffelement (3) und das untere Packstoffelement (4) im Bereich der Siegelfläche bzw. der Siegelnaht (5) jeweils mindestens einen Schnitt (7, 8) aufweisen, die deckungsgleich sind, und
- mindesten ein Schnitt (7, 8) durch eine Falz- oder Knicklinie (10) gekreuzt wird, **dadurch gekennzeichnet,**
**dass** jedes Packstoffelement (3, 4) eine äußere Schicht (11, 12) und eine Metallschicht (13, 14) und mindestens eine der beiden Packstoffelemente (3, 4) eine Siegelschicht (9) aufweist, und nur die äußere Schicht (11, 12) an der Falz- oder Knicklinie (10) geschwächt ausgebildet ist.

2. Verpackung (1) nach Anspruch 1,
**dadurch gekennzeichnet,**
**dass** die Falz- oder Knicklinie (10) durch perforieren, kerbstanzen, rillen oder durch Laserablation hergestellt ist.

3. Verpackung (1) nach Anspruch 1,
**dadurch gekennzeichnet,**
**dass** die Falz- oder Knicklinie (10) eine unterbrochene Linie ist.

4. Verpackung (1) nach Anspruch 1,
**dadurch gekennzeichnet,**
**dass** die Siegelfläche bzw. die Siegelnaht (5) nicht peelbar ist.

## Claims

1. A pack (1) for single-dose active-substance-containing films (2), comprising an upper packaging-material element (3) and a lower packaging-material element (4), which are interconnected by means of a peripheral sealing surface or sealing seam (5) in such a way that a cavity (6) that is enclosed on all sides and that is intended to receive the film (2) is formed, wherein
- the upper packaging material element (3) and the lower packaging material element (4) each have at least one cut (7, 8) in the region of the sealing surface or the sealing seam (5), said cuts being congruent, and
- at least one cut (7, 8) is intersected by a folding line or bending line (10),
**characterised in that**
each packaging material element (3, 4) has an outer layer (11, 12) and a metal layer (13, 14) and at least one of the two packaging elements (3, 4) has a sealing layer (9), and only the outer layer (11, 12) is formed on the folding line or bending line (10) in a weakened manner.

2. The pack (1) according to claim 1,
**characterised in that**
the folding line or bending line (10) is produced by perforation, punching of indentations, scoring or by laser ablation.

3. The pack (1) according to claim 1,
**characterised in that**
the folding line or bending line (10) is an interrupted line.

4. The pack (1) according to claim 1,
**characterised in that**
the sealing surface or the sealing seam (5) is not peelable.

## Revendications

1. Emballage (1) pour des films (2) unidoses contenant une substance active, comprenant un élément de matériau d'emballage supérieur (3) et un élément de matériau d'emballage inférieur (4), qui sont reliés ensemble au moyen d'une surface de scellement périphérique, respectivement, d'une jointure de scellement (5), de sorte qu'une cavité (6) fermée de tous les côtés est formée pour la réception du film (2), où
- l'élément de matériau d'emballage supérieur (3) et l'élément de matériau d'emballage inférieur (4) présentent dans la région de la surface de scellement, respectivement, de la jointure de scellement (5), chaque fois au moins une entaille (7, 8), coïncidant l'une avec l'autre, et
- au moins une entaille (7, 8) est croisée par une ligne de pliage ou de repliement (10), **caractérisé en ce que** chaque élément de matériau d'emballage (3, 4) présente une couche extérieure (11, 12) et une couche métallique (13, 14) et au moins l'un des deux éléments de matériau d'emballage (3, 4) présente une couche de scellement (9), et uniquement la couche extérieure (11, 12) est conçue fragilisée sur la ligne de pliage ou de repliement (10).

2. Emballage (1) selon la revendication 1,
**caractérisé en ce**
**que** la ligne de pliage ou de repliement (10) est fabriquée par perforation, par estampage de l'entaille, par rainurage ou par ablation laser.

3. Emballage (1) selon la revendication 1
**caractérisé en ce**
**que** la ligne de pliage ou de repliement (10) est une ligne discontinue.

4. Emballage (1) selon la revendication 1,
**caractérisé en ce**
**que** la surface de scellement, respectivement la jointure de scellemenFt (5), ne peut pas être pelée.
